(19) Europäisches Patentamt
European Patent Office
Office européen des brevets

(11) Numéro de publication: 0 053 532
B1

# (12) FASCICULE DE BREVET EUROPEEN

(45) Date de publication du fascicule du brevet: **09.11.83**

(51) Int. Cl.³: **B 01 J 23/85**, B 01 J 23/88

(21) Numéro de dépôt: **81401799.2**

(22) Date de dépôt: **16.11.81**

## (54) Procédé pour la préparation de catalyseurs à base d'oxydes de molybdène et/ou de tungstène et d'oxydes d'autres métaux.

(30) Priorité: **28.11.80 FR 8025261**

(43) Date de publication de la demande: **09.06.82 Bulletin 82/23**

(45) Mention de la délivrance du brevet: **09.11.83 Bulletin 83/45**

(84) Etats contractants désignés: **BE CH DE FR GB IT LI NL SE**

(56) Documents cités:
**FR - A - 1 600 128**
**FR - A - 1 604 942**
**FR - A - 2 322 855**
**FR - A - 2 360 347**
**FR - A - 2 364 061**
**FR - A - 2 441 423**
**US - A - 3 232 887**
**US - A - 4 070 397**

(73) Titulaire: **RHONE-POULENC CHIMIE DE BASE, 25, quai Paul Doumer, F-92408 Courbevoie (FR)**

(72) Inventeur: **Derrien, Jean-Yves, 9, rue Cécile Vallet, F-92340 Bourg-la-Reine (FR)**
Inventeur: **Belon, Paul, 27A, boulevard Gambetta, F-30100 Ales (FR)**

(74) Mandataire: **Dutruc-Rosset, Marie-Claude et al, RHONE POULENC RECHERCHES Service Brevets Chimie et Polymères 25, Quai Paul Doumer, F-92408 Courbevoie Cedex (FR)**

**EP 0 053 532 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

# Procede pour la preparation de catalyseurs a base d'oxydes de molybdene et/ou de tungstene et d'oxydes d'autres metaux

La présente invention a pour objet un perfectionnement à la préparation de catalyseurs. Elle concerne plus particulièrement la préparation de catalyseurs à base d'oxydes de molybdène, et/ou de tungstène.

De tels catalyseurs sont connus dans l'art antérieur. Ils sont utilisés, en particulier, pour la préparation d'aldéhydes $\alpha$-$\beta$ insaturés par oxydation d'oléfines en phase gazeuse.

C'est ainsi que le brevet français n° 1 514 167 décrit des catalyseurs pour la préparation d'aldéhydes insaturés par oxydation d'oléfines telles que le propylène et l'isopropylène par l'air ou l'oxygène de l'air répondant à la formule générale:

$$Ni_a Co_b Fe_c Bi_d As_e P_f Mo_g O_h$$

avec a compris entre 0 et 20, b entre 0 et 20, (a + b entre 0,5 et 20), c entre 0,5 et 8, d entre 0,1 et 7, e entre 0 et 3, f inférieur à 0,1, g égal à environ 12, h compris entre 36 et 98.

Ces catalyseurs sont préparés en ajoutant une solution aqueuse de sels solubles dans l'eau convenables de nickel, cobalt, fer et bismuth, d'un composé d'arsenic convenable et d'un composé du phosphore convenable à une solution aqueuse d'un molybdate convenable tel que le molybdate d'ammonium. La bouillie résultante est alors chauffée, avec un support si on le désire, pour éliminer l'eau et sécher le gâteau solide qui se forme. Le gâteau solide est ensuite calciné à une température élevée dans l'air. Des sels solubles dans l'eau convenables, mentionnés dans le brevet précité, sont le nitrate de nickel, le nitrate de cobalt, le nitrate ferrique, et le nitrate de bismuth par exemple.

Le brevet français n° 1 604 942 se rapporte à un procédé de préparation d'acroléine qui consiste à oxyder le propylène en phase gazeuse, avec un gaz contenant de l'oxygène et de la vapeur d'eau, en présence d'un catalyseur à base d'oxydes de molybdène, de bismuth, de fer et de cobalt dont la teneur en atome est la suivante: Mo 40,0 à 67,7%; Bi 1,9 à 21,7%; Fe 1,6 à 6,5% et Co 21,0 à 48,1%.

Le procédé de préparation de ces catalyseurs décrit dans ce brevet consiste à ajouter dans une solution aqueuse de molybdate d'ammonium vigoureusement agitée une solution aqueuse de nitrate de fer, cobalt et bismuth. Ensuite, on élimine l'eau et on calcine le produit résultant éventuellement sur un support.

Un autre document de l'art antérieur, la demande de brevet français n° 7 220 810, publiée sous le numéro 2 147 933 fait état d'un procédé de préparation de composés à groupe carbonyle insaturés à partir d'oléfines. Selon ce procédé, on conduit l'oxydation catalytique en présence d'un oxyde catalytique dans lequel les rapports atomiques des éléments catalytiques constitutifs Co/Fe/Bi/W/Mo/Si/Tl/Z (Z = métaux alcalins et alcalino-terreux) sont 2,0 à 20,0/0,1 à 10,0/0,1 à 10,0/0,5 à 10,0/2,0 à 11,5/0,5 à 15,0/0,005 à 3,0/0 à 3,0 sous réserve que W + Mo soit égal à 12,0.

On peut préparer ces catalyseurs en mélangeant des solutions aqueuses de molybdate d'ammonium et de p-tungstate d'ammonium, en ajoutant au mélange les solutions aqueuses de nitrate de cobalt, nitrate ferrique, nitrate de bismuth, nitrate de thallium, ainsi que la solution aqueuse d'un hydroxyde ou d'un nitrate d'un métal alcalin ou alcalino-terreux. On évapore ensuite l'eau et on calcine le produit obtenu éventuellement sur support.

On connait encore dans l'art antérieur, la demande de brevet d'invention n° 7 627 531, publiée sous le numéro 2 364 061 qui décrit un catalyseur à base d'oxyde de cobalt, de molybdène, de bismuth et de fer de formule $Co_a Mo_{12} Fe_b Bi_c O_x$ avec a compris entre 8 et 10, b entre 0,5 et 2, c entre 0,5 et 2 et x satisfaisant aux valences dans lequel la phase active contient une phase répondant à la formule $Bi_2 Mo_2 Fe_2 O_{12}$. Ces catalyseurs permettent d'obtenir des rendements très sensiblement améliorés lors, par exemple, de l'oxydation d'oléfines en aldéhydes $\alpha$-$\beta$ insaturés.

On prépare ces catalyseurs en faisant réagir une solution d'heptamolybdate d'ammonium et une solution de nitrates de Co, Bi et Fe. On évapore ensuite l'eau et on sèche la pâte. Le solide obtenu est soumis après une éventuelle pré-calcination à environ 450° C à une première calcination à une température comprise entre 450° C et 500° C pendant au moins 5 heures puis, après refroidissement, à température ambiante à une deuxième calcination dans les mêmes conditions que la première.

On constate donc que dans l'art antérieur, il existe un grand nombre de documents décrivant la mise en œuvre pour l'oxydation par l'air d'oléfines — et, en particulier, pour l'oxydation de propylène en acroléine — de catalyseurs à base principalement d'oxydes de cobalt, fer, bismuth et molybdène. Un certain nombre d'oxydes d'autres métaux ont été proposés comme adjuvants à la composition de base. On constate également que les procédés de préparation de ces catalyseurs font tous intervenir dans la première étape, la réaction entre l'heptamolybdate d'ammonium et les nitrates de fer, cobalt et bismuth.

Il se forme lors de cette réaction du nitrate d'ammonium qui se décomposera thermiquement à environ 220° C lors d'une calcination postérieure.

Il est clair que cette décomposition thermique du nitrate d'ammonium pose de sérieux problèmes pratiques. En effet, l'homme de l'art sait bien que le nitrate d'ammonium est un composé explosif et, à ce titre, est d'une manipulation à l'échelle industrielle dangereuse.

Il est à souligner que dans la pratique

industrielle, il est extrêmement difficile, sinon impossible, de s'affranchir de la présence simultanée de sels d'ammonium et de nitrates.

On constate donc que subsiste dans l'art antérieur le besoin d'un procédé permettant la préparation de catalyseurs à base notamment d'oxydes de molybdène et/ou de tungstène, faisant intervenir la réaction d'au moins un sel d'ammonium et d'au moins un nitrate de ces métaux dans lequel il n'y ait pas de risque, lors des opérations ultérieures de calcination, de décomposition thermique du nitrate d'ammonium.

La demanderesse a découvert un procédé atteignant cet objectif.

La présente invention a donc pour objet un procédé de préparation de catalyseurs comportant une phase active répondant à la formule générale:

$$A_aMo_cW_dCo_eB_bO_x$$

dans laquelle:

- A représente au moins un métal choisi parmi le groupe comprenant le nickel, le manganèse, le plomb,
- B représente au moins un métal choisi parmi le groupe comprenant le fer, le bismuth, le chrome, le thallium,
- a représentant la somme des indices affectés à chacun des métaux A, est supérieur ou égal à 0 et inférieur ou égal à 4
- c et d sont chacun supérieurs ou égaux à 0 et inférieurs ou égaux à 12, leur somme c + d étant égale à environ 12;
- e est supérieur ou égal à 8 et inférieur ou égal à 12
- b représentant la somme des indices affectés à chacun des métaux B, est supérieur à 0 et inférieur ou égal à environ 1,5[(c + d) − (a + e)]
- et x satisfait aux valences

procédé du type selon lequel dans une première étape, on mélange en phase aqueuse des sels des métaux entrant dans la composition de la phase active, le molybdène et/ou le tungstène étant sous forme de sels d'ammonium et au moins l'un des métaux A et B étant sous forme d'un nitrate, dans une deuxième étape on sèche la pâte obtenue et dans une troisième étape, on effectue au moins une calcination du solide obtenu éventuellement déposé sur un support, procédé caractérisé en ce que dans la première étape, l'on acidifie une première solution aqueuse contenant les sels d'ammonium de molybdène et de tungstène en quantités permettant d'atteindre les valeurs c et d jusqu'à un pH compris entre 0,5 et 3; l'on sépare le précipité obtenu que l'on met ensuite en suspension dans de l'eau, de façon à obtenir une suspension ayant un pH compris entre 1,5 et 2,5; l'on ajoute à la suspension une deuxième solution aqueuse contenant les sels des métaux A et B en quantités permettant d'atteindre les valeurs a et b; puis, ultérieurement, l'on ajoute du carbonate de cobalt sous forme de poudre en quantité permettant d'atteindre la valeur e; et l'on évapore la solution obtenue pour obtenir la dite pâte.

Le procédé selon l'invention est basé sur le fait que quand on acidifie la solution contenant les sels d'ammonium de molybdène et/ou de tungstène, de l'acide molybdique et/ou tungstique précipite et les ions ammonium restent en solution; ces derniers peuvent être éliminés par filtration par exemple. Les ions ammonium éliminés de façon quasi-quantitative, il n'y a plus de risque de voir se former dans la suite du procédé, du nitrate d'ammonium. Mais la suspension d'acide molybdique et/ou tungstique est acide (son pH est compris entre 1,5 et environ 2). Si alors on ajoute les autres métaux (cobalt, A et B) tous sous forme de nitrates, on abaisse encore le pH qui peut devenir inférieur à 0,5. A ce pH, on n'observe pas une précipitation satisfaisante des métaux, en particulier du fer, qui repasse en solution avec le molybdène.

Pour éviter ce problème, il faut avoir un pH voisin de 2 environ. Il serait possible de rajouter de l'ammoniaque mais alors on en revient au problème de départ puisque du nitrate d'ammonium se formerait. La demanderesse a découvert qu'il fallait ajouter le métal majoritaire autre que le molybdène et/ou le tungstène, c'est-à-dire le cobalt, sous forme d'un sel décomposable par l'acidité du milieu. Le carbonate de cobalt répond à cette définition.

On peut citer plus particulièrement comme sel d'ammonium de molybdène pouvant être utilisé dans le cadre du procédé selon l'invention, l'heptamolybdate d'ammonium. Ce dernier peut être mis en œuvre sous forme d'heptamolybdate d'ammonium cristallisé, soit sous forme d'un mélange de dimolybdate d'ammonium et d'anhydride molybdique.

Lorsque la première solution aqueuse contient uniquement de l'heptamolybdate d'ammonium sous une des formes précitées, son pH est de 5,4 environ.

On peut citer plus particulièrement comme sel d'ammonium de tungstène, le paratungstate d'ammonium.

Lorsque la première solution aqueuse contient uniquement du paratungstate d'ammonium, son pH est de 5,8 environ.

Lorsqu'on a un mélange et paratungstate d'ammonium et d'heptamolybdate d'ammonium, le pH oscille, suivant les proportions relatives des deux constituants, entre environ 5,4 et environ 5,8.

L'acidification de la première solution aqueuse se fait à l'aide d'un acide assez fort pour atteindre un pH compris entre 0,5 et 3. Il est préférable également que ce soit un acide dont l'anion soit facilement décomposable thermiquement. On peut citer comme exemple d'un tel

acide, l'acide nitrique.

Selon un mode de réalisation préférentiel de l'invention, on acidifie la première solution aqueuse jusqu'à obtention d'un pH compris entre 0,7 et 1,5.

Selon un autre mode de réalisation préférentiel de l'invention le pH de la suspension est égal à environ 2.

Selon un mode de mise en œuvre particulier de l'invention, on utilise une première solution aqueuse contenant uniquement de l'heptamolybdate d'ammonium, une deuxième solution aqueuse ne contenant pas de métaux A et contenant comme métaux B, le fer, le bismuth et du carbonate de cobalt en quantités permettant d'atteindre la formule catalytique où e est compris entre 8 et 10, c est égal à environ 12 et b est compris entre 1 et 4.

Encore plus précisément, le procédé selon l'invention est particulièrement adapté à la mise en œuvre des mêmes solutions et carbonate de cobalt, en quantités permettant d'atteindre la formule catalytique où e est égal à environ 10, c est égal à environ 12 et b égal à environ 2.

Les diverses opérations du procédé selon l'invention sont faites à température ambiante de préférence.

L'évaporation finale se fait de préférence à une température comprise entre environ 70° C et 100° C.

Les opérations ultérieures de séchage et de calcination(s) éventuellement sur support se font selon les techniques décrites dans l'art antérieur.

L'invention va être maintenant plus complètement décrite à l'aide des exemples qui vont suivre. Ces derniers ne sauraient être interprétés comme constituant une limitation quelconque de l'invention.

### Exemple 1

Préparation de $Co_{10} Mo_{12} Fe_1 Bi_1 O_x$

On prépare une première solution aqueuse d'heptamolybdate d'ammonium en dissolvant 167,6 g de $(NH_4)_6 Mo_7 O_{24}, 4 H_2O$ dans 760 $cm^3$ d'$H_2O$ à température ambiante. On ajoute à cette solution en agitant un mélange de 72,5 $cm^3$ d'$HNO_3$ de densité 1,33 et de 72,5 $cm^3$ d'$H_2O$. Le pH de la solution résultante est de 0,8.

On filtre. On récupère un solide blanc cristallisé qui est de l'acide molybdique (contenant 93,8 g de $Mo O_3$).

Le taux de précipitation du molybdène est de 97%.

Les ions $NH_4^+$ de l'heptamolybdate d'ammonium se trouve dans le filtrat sous forme de nitrate d'ammonium. L'analyse du filtrat montre que la totalité des ions $NH_4^+$ ont été éliminés.

On met en suspension, ce précipité dans 600 ml d'eau. Le pH de la suspension obtenue est de 1,9.

On prépare une deuxième solution aqueuse en mélangeant:

- 38,4 g de Bi $(NO_3)_3$, 5 $H_2O$ dans 28,5 $cm^3$ d'$H_2O$ acidifiée par 4,5 $cm^3$ d'acide nitrique.
- 32,1 g de Fe $(NO_3)_3$, 9 $H_2O$ dans 25 $cm^3$ d'$H_2O$.

On additionne cette deuxième solution aqueuse à la suspension précédente en agitant. Le pH est alors de 0,6.

On introduit alors 94 g de Co $CO_3$ en poudre tout en agitant.

On observe un dégagement de $CO_2$. En fin d'addition, le pH est de 3,4. On poursuit l'agitation pendant 30 minutes.

On évapore l'eau en maintenant à 80° C pendant 3 heures. On obtient une pâte non coulante qui, traitée ensuite selon les techniques de l'art antérieur, donne un catalyseur éventuellement supporté, de formule $Co_{10} Mo_{12} Fe_1 Bi_1 O_x$

### Exemple 2

Préparation de $Co_8 Mo_{12} Fe_{0,5} Bi_{0,5} O_x$

On ajoute à la première solution aqueuse obtenue dans l'exemple 1, un mélange de 73 $cm^3$ d'$HNO_3$ (densité = 1,33) et de 146 $cm^3$ d'$H_2O$. Le pH final est de 1,3. On filtre; on récupère un solide blanc cristallin qui est de l'acide molybdique contenant 95,2 g de $Mo O_3$. Le taux de précipitation du molybdène est de 98,5%.

Tous les ions $NH_4^+$ se trouvent dans le filtrat sous forme de nitrate d'ammonium.

On met en suspension ce précipité dans 750 ml d'$H_2O$. Le pH est de 1,8.

On prépare une deuxième solution aqueuse en mélangeant:

- 19,2 g de Bi $(NO_3)_3$, 5 $H_2O$ dans 28,5 $cm^3$ d'$H_2O$ acidifiée par 4,5 $cm^3$ d'acide nitrique
- 16 g de Fe $(NO_3)_3$, 9 $H_2O$ dans 25 $cm^3$ d'$H_2O$.

On additionne cette dexième solution aqueuse à la suspension précédente en agitant. Le pH est alors de 1,3.

On introduit alors 75,1 g de carbonate de cobalt en poudre tout en agitant. On observe un dégagement de $CO_2$. En fin d'addition le pH est de 2,8. On poursuit l'agitation pendant 30 minutes.

On évapore à 80°C pendant 3 heures. On obtient une âte non coulante qui traitée selon les techniques de l'art antérieur, donne un catalyseur éventuellement supporté, de formule:

$Co_8 Mo_{12} Fe_{0,5} Bi_{0,5} O_x$

## Revendications

1. Procédé de préparation de catalyseurs comportant une phase active répondant à la formule générale:

$A_a Mo_c W_d Co_e B_b O_x$

dans laquelle:

- A représente au moins un métal choisi parmi le groupe comprenant le nickel, le manganèse, le plomb,
- B représente au moins un métal choisi parmi le groupe comprenant le fer, le bismuth, le chrome, le thallium,
- a représentant la somme des indices affectés à chacun des métaux A, est supérieur ou égal à 0 et inférieur ou égal à 4
- c et d sont chacun supérieurs ou égaux à 0 et inférieurs on égaux à 12, leur somme c+d étant égale à environ 12;
- e est supérieur ou égal à 8 et inférieur ou égal à 12;
- b représentant la somme des indices affectés à chacun des métaux B, est supérieur à 0 et inférieur ou égal à 1,5 [(c+d)−(a+e)]
- et x satisfait aux valences

procédé du type selon lequel dans une première étape on mélange en phase aqueuse des sels des métaux entrant dans la composition de la phase active, le molybdène et/ou le tunstène étant sous forme de sels d'ammonium et au moins l'un des métaux A et B étant sous forme d'un nitrate, dans une deuxième étape on sèche la pâte obtenue et dans une troisième étape on effectue au moins une calcination du solide obtenu éventuellement déposé sur un support, procédé caractérisé en ce que dans la première étape, l'on acidifie une première solution aqueuse contenant les sels d'ammonium de molybdène et/ou de tungstène en quantités permettant d'atteindre les valeurs c et d jusqu'à un pH compris entre 0,5 et 3; l'on sépare le précipité obtenu que l'on met ensuite en suspension dans de l'eau de façon à obtenir une suspension ayant un pH compris entre 1,5 et 2,5; l'on ajoute à la suspension une deuxième solution aqueuse contenant les sels des métaux A et B en quantités permettant d'atteindre les valeurs a et b; puis, ultérieurement, l'on ajoute du carbonate de cobalt sous forme de poudre en quantité permettant d'atteindre la valeur e; et l'on évapore la solution obtenue pour obtenir la dite pâte.

2. Procédé selon la revendication 1 caractérisé en ce que la première solution aqueuse contient uniquement de l'heptamolybdate d'ammonium.

3. Procédé selon la revendication 1 caractérisé en ce que la première solution aqueuse contient uniquement du paratungstate d'ammonium.

4. Procédé selon la revendication 1 caractérisé en ce que la première solution aqueuse contient un mélange d'heptamolybdate d'ammonium et de paratungstate d'ammonium;

5. Procédé selon la revendication 1 caractérisé en ce que l'on acidifie la première solution aqueuse avec de l'acide nitrique.

6. Procédé selon la revendication 1 ou 5 caractérisé en ce que l'on acidifie la première solution aqueuse jusqu'à un pH compris entre 0,7 et 1,5.

7. Procédé selon la revendication 1 caractérisé en ce que le pH de la suspension est égal à environ 2.

8. Procédé selon la revendication 1 caractérisé en ce que l'on utilise une première solution aqueuse contenant uniquement de l'heptamolybdate d'ammonium, une deuxième solution aqueuse ne contenant pas de métaux A et contenant comme métaux B, le fer et le bismuth et du carbonate de cobalt en quantité permettant d'atteindre la formule catalytique où e est compris entre 8 et 10, c est égal à environ 12 et b est compris entre 1 et 4.

9. Procédé selon la revendication 8 caractérisé en ce que e est égal à environ 10 et b est égal à environ 2.

**Patentansprüche**

1. Verfahren zur Herstellung von Katalysatoren enthaltend eine aktive Phase der allgemeinen Formel

$A_a Mo_c W_d Co_e B_b O_x$

in der

- A wenigstens ein Metall aus der Gruppe von Nickel, Mangan und Blei
- B wenigstens ein Metall aus der Gruppe von Eisen, Wismut, Chrom und Tahllium
- a die Summe der jedem der Metalle A entsprechenden Indizes bedeutet und größer oder gleich 0 und kleiner oder gleich 4 ist
- c und d jeweils größer oder gleich 0 und kleiner oder gleich 12 sind, wobei die Summe c+d ungefähr gleich 12 ist;
- e größer oder gleich 8 und kleiner oder gleich 12 ist;
- b die Summe der zu den Metallen B gehörenden Indizes bedeutet und größer als 0 und kleiner oder gleich 1,5[(c+d)−(a+e)] ist und
- x den Valenzen genügt

bei dem man in einer ersten Stufe in wäßriger Phase Salze der Metalle gemäß der Zusammensetzung der aktiven Phase vermischt, wobei das Molybdän und/oder das Wolfram in Form der Ammoniumsalze und wenigstens eines der Metalle A und B in Form eines Nitrats vorliegen, in einer zweiten Stufe die erhaltene Paste trocknet und in einer dritten Stufe den gegebenenfalls auf einem Träger aufgebrachten Feststoff wenigstens einer Calcinierung unterwirft, dadurch gekennzeichnet, daß man in der ersten Stufe eine erste wäßrige Lösung, die die Ammoniumsalze des Molybdäns und/oder des Wolframs in einer zur Erreichung der Werte c

und d ausreichenden Menge enthält, bis auf einen pH-Wert zwischen 0,5 und 3 ansäuert, den erhaltenen Niederschlag abtrennt und dann in Wasser suspendiert, um eine Suspension mit einem pH-Wert zwischen 1,5 und 2,5 zu erhalten, dieser Suspension eine zweite wäßrige Lösung zusetzt, die die Salze der Metalle A und B in den zur Erreichung der Werte a und b erforderlichen Mengen enthält und schließlich pulverförmiges Kobaltcarbonat in der zur Erreichung des Werts e erforderlichen Menge zugibt und die erhaltene Lösung eindampft, um die genannte Paste zu erhalten.

2. Verfahren nach Anspruch 1, dadurch gekennzeichnet, daß die erste wäßrige Lösung nur Ammoniumheptamolybdat enthält.

3. Verfahren nach Anspruch 1, dadurch gekennzeichnet, daß die erste wäßrige Lösung nur Ammoniumparawolframat enthält.

4. Verfahren nach Anspruch 1, dadurch gekennzeichnet, daß die erste wäßrige Lösung ein Gemisch von Ammoniumheptamolybdat und Ammoniumparawolframat enthält.

5. Verfahren nach Anspruch 1, dadurch gekennzeichnet, daß man die erste wäßrige Lösung mit Salpetersäure ansäuert.

6. Verfahren nach Anspruch 1 oder 5, dadurch gekennzeichnet, daß man die erste wäßrige Lösung bis zu einem pH zwischen 0,7 und 1,5 ansäuert.

7. Verfahren nach Anspruch 1, dadurch gekennzeichnet, daß der pH-Wert der Suspension etwa 2 beträgt.

8. Verfahren nach Anspruch 1, dadurch gekennzeichnet, daß man eine erste wäßrige Lösung, die nur Ammoniumheptamolybdat enthält, eine zweite wäßrige Lösung, die keine Metalle A und als Metall B Eisen und Wismut enthält und ferner Kobaltcarbonat in der Menge verwendet, die zur Erreichung der Katalysatorformel erforderlich ist, in der e gleich 8 bis 10, c etwa 12 und b gleich 1 bis 4 ist.

9. Verfahren nach Anspruch 8, dadurch gekennzeichnet, daß e etwa 10 und b etwa 2 ist.

## Claims

1. A process for the preparation of catalysts comprising an active phase corresponding to the general formula:

$$A_a Mo_c W_d Co_e B_b O_x$$

in which:

- A represents at least one metal selected from the group comprising nickel, manganese and lead,
- B represents at least one metal selected from the group comprising iron, bismuth, chromium and thallium,
- a representing the sum of the indexes associated with each of the metals A is greater than or equal to 0 an lower than or equal to 4,
- c and d are each greater than or equal to 0 and lower than or equal to 12, the sum thereof c+d being equal to about 12,
- e is greater than or equal to 8 and lower than or equal to 12,
- b representing the sum of the indexes associated with each of the metals B is greater than 0 and lower than or equal to 1.5 [(c+d)−(a+e)], and
- x satisfies the valencies,

being a process of the type wherein, in a first stage, salts of the metals involved in the composition of the active phase are mixed in the aqueous phase, molybdenum and/or tungsten being in the form of ammonium salts and at least one of the metals A and B being in the form of a nitrate, in a second stage, the resulting paste is dried and, in a third stage, at least one operation of calcination of the solid produced, which is possibly deposited on a carrier, is effected, characterised in that, in the first stage, acidification is effected in respect of a first aqueous solution containing the molybdenum and/or tungsten ammonium salts in amounts which make it possible to attain the values c and d, to a pH-value thereof of between 0.5 and 3; the resulting precipitate is separated and then put in suspension in water so as to produce a suspension having a pH-value of trom 1.5 to 2.5; added to the suspension is a second aqueous solution containing the salts of the metals A and B in quantities making it possible to attain the values a and b; subsequently, cobalt carbonate is added in powder form, in an amount which makes it possible to attain the value e; and the resulting solution is evaporated to produce said paste.

2. A process according to claim 1 characterised in that the first aqueous solution contains only ammonium heptamolybdate.

3. A process according to claim 1 characterised in that the first aqueous solution contains only ammonium paratungstate.

4. A process according to claim 1 characterised in that the first aqueous solution contains a mixture of ammonium heptamolybdate and ammonium paratungstate.

5. A process according to claim 1 characterised in that the first aqueous solution is acidified with nitric acid.

6. A process according to claim 1 or claim 5 characterised that the first aqueous solution is acidified until it is of a pH-value of between 0.7 and 1.5.

7. A process according to claim 1 characterised in that the pH-value of the suspension is equal to about 2.

8. A process according to claim 1 characterised by using a first aqueous solution containing only ammonium heptamolybdate, a second aqueous solution which does not contain metals A and which contains, as metals B, iron and bismuth, and cobalt carbonate in an amount

making it possible to attain the catalytic formula in which e is between 8 and 10, c is equal to about 12 and b is between 1 and 4.

9. A process according to claim 8 characterised in that e is equal to about 10 and b is equal to about 2.